# EUROPEAN PATENT SPECIFICATION

(11) **EP 4 280 975 B1**
(45) Date of publication and mention of the grant of the patent: **30.07.2025**
(21) Application number: 22829888.1
(22) Date of filing: 14.12.2022
(51) Int. Cl.: A61B 17/04, A61B 17/34, A61B 17/00

(54) **SUTURE NEEDLE GUIDE TUBE AND TISSUE SUTURING DEVICE**
NAHTNADELFÜHRUNGSROHR UND GEWEBENAHTVORRICHTUNG
TUBE DE GUIDAGE D'AIGUILLE DE SUTURE ET DISPOSITIF DE SUTURE DE TISSU

(30) Priority: 17.12.2021 CN 202111553006
(43) Date of publication of application: 29.11.2023
(73) Proprietor: Cilag GmbH International, 6300 Zug (CH)
(72) Inventor: ZHANG, Jie, Shanghai 200233 (CN); DING, Sheng, Shanghai 200233 (CN); LIU, Qinglong, Shanghai 200233 (CN)
(74) Representative: van Wanrooij, Eva
(86) International application number: PCT/IB2022/062192
(87) International publication number: WO 2023/111881

(56) References cited:
- EP-A1- 3 222 220
- EP-A2- 3 420 985
- US-A1- 2017 231 621

## Description

### TECHNICAL FIELD

The present invention relates to the technical field of surgical instruments, in particular, to a suture needle guide tube and a tissue suturing device.

### BACKGROUND OF THE INVENTION

A surgeon performing surgery generally needs to use a tissue closure instrument to suture a wound to be sutured of a patient, such as an incision of the abdominal cavity, etc.

When an existing tissue closure instrument is used to perform suturing, a puncture needle needs to be used to puncture tissue. An experienced surgeon can use the puncture needle with experience to puncture properly without deviation. However, for many surgeons, a deviated puncture often occurs due to shaking of a puncture needle, and therefore a plurality of punctures need to be performed. Although an anesthetic has been injected to an incision situs of a patient, a plurality of punctures increase injury to tissue at the incision of the patient.

Additionally, in endoscopic surgery, a surgeon establishes a channel via a puncture cannula. The channel allows some surgical instruments to pass therethrough to perform related surgical operations. After completing the surgery, an incision needs to be sutured. Therefore, the puncture cannula needs to be pulled out, and then a tissue closure instrument needs to be inserted to suture the incision. In actual operation, however, the tissue closure instrument may not be capable of being subjected to such an insertion operation again along the original incision due to various factors. Therefore, a new wound may be made in the tissue, and the new wound is sutured. Such a suturing position is not the correct suturing position of the original incision.

Additionally, it is not easy to use an existing tissue closure instrument to accurately arrange a suture in a desired position in an inner cavity, so that some surgeons need to shake a puncture needle a plurality of times to search for the suture. However, such shaking damages a puncture in tissue to some extent.

US 2017/231621 A1 discloses apparatus and methods for treating a tissue opening, for example a trocar opening used in a minimally invasive surgical procedure. Introducers can be used having a suture holder, an alignment indicator and insertion limits. EP 3 420 985 A2 discloses a surgical access device which includes a cannula and a housing coupled to the cannula. A cannula lumen and a housing interior communicate to define a working channel that extends along a central axis of the device between proximal and distal device ends. A needle entrance port is arranged on a side portion of the housing and opens to the working channel. A needle exit port is arranged distally of the needle entrance port on a side portion of the cannula, and communicates with the needle entrance port to define a suture path extending through the surgical access device at an oblique angle relative to the central axis. The needle ports are configured to guide a suture passer device distally through the surgical access device and first and second tissues of different thicknesses, while maintaining the same tissue bite distance in each of the first and second tissues. EP 3 222 220 A1 discloses a surgical suture for a laparoscopic port site closure device. The surgical suture is configured such that a cartridge receiving surgical suture therein is provided at a fore-end of a needle guide for port site closure; the surgical suture is caught in a needle tip that is pierced through a body tissue by being guided by the needle guide; and when the needle is withdrawn, the surgical suture is pulled out along a path through which the needle was pierced into the tissue, and the surgical suture is tied outside a patient's body, thereby being capable of closing a laparoscopic port site. The laparoscopic port site closure device is in a tube shape for being introduced into a port site, and includes: a tubular body provided with needle guides that face each other and guide insertion of a needle; wings mounted to a lower portion of the tubular body such that the wings are opened and closed through a cam method; and an operating stick penetrating through the tubular body to operate the wings by being rotated to push and open the wings and to pull and close the wings by being rotated reversely.

### SUMMARY

The present invention provides a suture needle guide tube according to claim 1. Optional features are recited in the dependent claims. The suture needle guide tube can guide a suture needle to puncture accurately in a predetermined direction, and a deviated puncture caused by shaking of the suture needle is avoided, thereby reducing injury to tissue of a wound to be sutured of a patient.

In the technical solution, the tube body is capable of being inserted into a wound to be sutured, and the tube body comprises the internal axial channel for arranging the handling tube provided with the suture arrangement member and the handling member. In this manner, during suturing, the tube body does not need to be pulled out of the wound or an incision, and the handling tube only needs to be inserted into the internal axial channel of the tube body, so that the suture arrangement member only needs to arrange a suturing member in an inner cavity, thereby avoiding additional tissue injury caused by a mistake made in the second puncture. The suture needle guide is provided on the tube body, and the suture needle passing path on the tube body correspondingly communicates with the suture needle guide channel on the suture needle guide. In this manner, effective guiding performed by the suture needle guide channel prevents shaking, so that the suture needle can pass through the suture needle passing path stably, so as to pass through parietal tissue of the inner cavity and then enter the inner cavity so as to hook or hold an arranged suture. Additionally, after the suture needle hooks or holds the suture, effectively guided by the suture needle guide channel, the suture needle with the suture can stably pass through the parietal tissue and then exit. Therefore, the suture needle guide tube can guide the suture needle to puncture accurately in a predetermined direction, and a deviated puncture caused by shaking of the suture needle is avoided, thereby reducing injury to tissue of a wound to be sutured of a patient. Furthermore, the internal axial channel of the tube body may provide an access channel for other surgical instruments.

The present invention also provides a tissue suturing device according to claim 6. Optional features are recited in the dependent claims. The tissue suturing device not only can guide a suture needle to puncture accurately in a predetermined direction to prevent a deviated puncture caused by shaking of the suture needle, but also can accurately arrange a suture in a desired position, so that the suture can be led out after the suture needle punctures once, thereby significantly reducing injury to tissue of a wound to be sutured of a patient.

In the technical solution, the suture needle path on the sleeve tube main body can effectively guide the suture needle to pass therethrough, for example, vertically or obliquely. In this manner, the suture needle can be prevented from shaking during a puncture, so that the suture needle can pass through the suture needle passing path stably, so as to pass through parietal tissue of an inner cavity and then enter the inner cavity. Additionally, the handling member drives, by means of the transmitting member, the suture arrangement member to change, in the inner cavity, from the folded position to the unfolded position, so that the suture is accurately extended to and arranged on two sides of the wound to be sutured in the inner cavity. After the suture needle having entered the inner cavity holds the suture, effectively guided by the suture needle path on the sleeve tube main body, the suture needle with the suture can stably pass through the parietal tissue and then exit. Therefore, the tissue suturing device not only can guide a suture needle to puncture accurately in a predetermined direction to prevent a deviated puncture caused by shaking of the suture needle, but also can accurately arrange a suture in a desired position, so that the suture can be led out after the suture needle punctures once, thereby significantly reducing injury to tissue of a wound to be sutured of a patient.

### BRIEF DESCRIPTION OF THE DRAWINGS

To better understand the above and other objectives, features, advantages, and functions of the present invention, reference may be made to the preferred embodiments shown in the accompanying drawings. The same or similar reference numerals in the accompanying drawings refer to the same or similar components. It should be understood by those skilled in the art that the accompanying drawings are intended to schematically illustrate preferred embodiments of the present invention, instead of setting any limitations on the scope of the present invention. The various components in the drawings are not drawn to scale.
FIG. 1 is a schematic perspective structural view of a sleeve tube main body of a tissue suturing device according to a specific embodiment of the present invention, wherein a suture needle guide tube provided in the specific embodiment of the present invention is shown.
FIG. 2 is a schematic side structural view of the sleeve tube main body in FIG. 1.
FIG. 3 is a schematic perspective structural view of a tube body of the suture needle guide tube in FIG. 1.
FIG. 4 is a schematic side structural view of the tube body in FIG. 3.
FIG. 5 is a schematic perspective structural view of a suture needle guide of the suture needle guide tube in FIG. 1.
FIG. 6 is a schematic perspective structural view of assembly of a handling member, a suture arrangement member, and a handling tube in a tissue suturing device according to a specific embodiment of the present invention.
FIG. 7 is a schematic partial structural view of FIG. 6, and shows a suture.
FIG. 8 is a schematic perspective structural view of a suture arrangement wing of a suture arrangement member in FIG. 6.
FIG. 9 is a schematic top structural view of the suture arrangement wing in FIG. 8.
FIG. 10 is a schematic perspective structural view of assembly of a handling member, a suture arrangement member, and a handling tube in another tissue suturing device according to a specific embodiment of the present invention, wherein the suture arrangement member is in a folded position.
FIG. 11 is a schematic side structural view of FIG. 10.
FIG. 12 is a schematic sectional structural view of a position in FIG. 11.
FIG. 13 is a schematic structural view of the suture arrangement member of FIG. 10 in an unfolded position.
FIG. 14 is a schematic sectional structural view of a position in FIG. 13.
FIG. 15 is a schematic partial structural view of FIG. 13, and shows a suture.
FIG. 16 is a schematic perspective structural view of a suture arrangement wing in FIG. 13.

### BRIEF DESCRIPTION OF THE DRAWINGS

1 - tube body, 2 - suture needle guide, 3 - suture needle guide channel, 4 - suture needle passing port, 5 - sleeve, 6 - oblique cylinder, 7 - protuberant piece, 8 - sleeve tube main body, 9 - handling member, 10 - transmitting member, 11 - suture arrangement member, 12 - suture arrangement wing, 13 - linkage, 14 - suture accommodating gap, 15 - suture needle guide tube, 16 - handling tube, 17 - suture receiving recess, 18 - vertical rod, 19 - transverse rod, 20 - cross, 21 - suture passing hole, 22 - suture, 23 - suture support column, 24 - suture bypassing column, 25 - suture needle suture-holding port.

### DETAILED DESCRIPTION

In the following detailed description of embodiments, illustration is provided with reference to the accompanying drawings that constitute a part of the description. The accompanying drawings illustrate, by means of examples, specific embodiments, and the present invention is implemented in these embodiments. The illustrated embodiments are not intended to be exhaustive embodiments of the present invention. It will be appreciated that other embodiments may be utilized, and changes in structures or logic may be made without departing from the scope of the present invention. For the accompanying drawings, directional terms, such as "lower," "upper," "left," "right," etc., are used with reference to the positions and directions in the accompanying drawings. Since the components of the embodiments of the present invention can be implemented in a variety of positions and directions, these directional terms are for illustrative purposes, rather than limiting purposes. Therefore, the following specific embodiments are not intended to set limitations, and the scope of the present invention is defined by the appended claims.

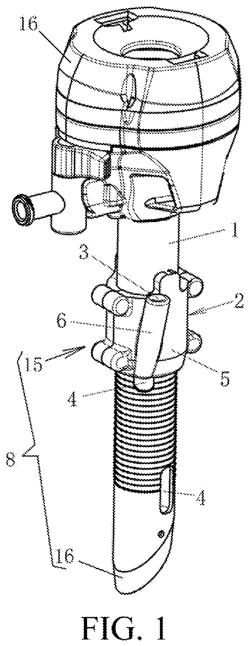

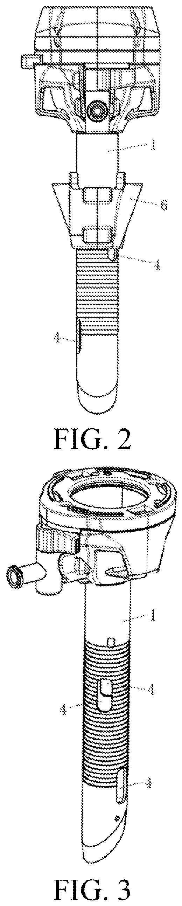

In a first aspect, referring to FIG. 1, FIG. 2, FIG. 3, FIG. 4, and FIG. 5, a suture needle guide tube 15 provided in a specific embodiment of the present invention includes a tube body 1 and a suture needle guide 2. The tube body 1 is for being inserted into a wound to be sutured. The tube body 1 includes an internal axial channel for arranging a handling tube provided with a suture arrangement member and a handling member. The tube body 1 is provided with a plurality of suture needle passing paths located in different positions for guiding a suture needle to pass therethrough. The suture needle guide 2 is provided with a plurality of suture needle guide channels 3 located in different positions. The suture needle guide 2 is provided on the tube body 1, and each suture needle guide channel 3 respectively communicates with the corresponding suture needle passing path so as to guide the suture needle to move out of the suture needle guide channel 3 and then pass through the suture needle passing path to enter an inner cavity and to allow the suture needle to exit with a suture.

In the suture needle guide tube 15, the tube body 1 is capable of being inserted into a wound to be sutured, and the tube body 1 includes the internal axial channel for arranging the handling tube provided with the suture arrangement member and the handling member. In this manner, during suturing, the tube body does not need to be pulled out of the wound or an incision, and the handling tube only needs to be inserted into the internal axial channel of the tube body, so that the suture arrangement member only needs to arrange a suturing member in an inner cavity, thereby avoiding additional tissue injury caused by a mistake made in the second puncture. The suture needle guide 2 is provided on the tube body 1, and the suture needle passing path on the tube body 1 correspondingly communicates with the suture needle guide channel 3 on the suture needle guide 2. In this manner, effective guiding performed by the suture needle guide channel 3 prevents the suture needle from shaking during a puncture, so that the suture needle can pass through the suture needle passing path stably, so as to pass through parietal tissue of the inner cavity and then enter the inner cavity so as to hook or hold an arranged suture. Additionally, after the suture needle hooks or holds the suture, effectively guided by the suture needle guide channel 3, the suture needle with the suture can stably pass through the parietal tissue and then exit, so that the suture is guided to pass through the wound to be sutured so as to suture the same. Therefore, the suture needle guide tube 15 can guide the suture needle to puncture accurately in a predetermined direction, and a deviated puncture caused by shaking of the suture needle is avoided, thereby reducing injury to tissue of a wound to be sutured of a patient. Furthermore, the internal axial channel of the tube body may provide an access channel for other surgical instruments.

In the suture needle guide tube 15, the suture needle guide 2 is capable of being adjusted to a predetermined position by means of axial movement along the tube body so that one suture needle guide channel 3 correspondingly communicates with one suture needle passing path in the predetermined position. For example, the tube body 1 is provided with a plurality of suture needle passing paths that are axially spaced apart from each other. Due to personal differences, the thickness of the tissue of the wound to be sutured may be different, so that the suture needle guide 2 can be adjusted to the predetermined position by means of the axial movement along the tube body according to the different thickness of the tissue. In the predetermined position, each suture needle guide channel 3 respectively correspondingly communicates with the corresponding suture needle passing path in the predetermined position, thereby better meeting wound suturing requirements of different patients.

Additionally, in the suture needle guide tube 15, the suture needle guide 2 can be held and positioned in the predetermined position by using a variety of methods. For example, in a method, a plurality of concave parts axially spaced apart from each other are formed on the tube body 1, and a positioning ball is connected to the suture needle guide 2 by means of an elastic member such as a spring. The positioning ball may slide into or out of each concave part. In this manner, after the suture needle guide 2 moves to the predetermined position, the positioning ball under the action of the elastic member slides into the concave part in the predetermined position, so that the suture needle guide 2 is held and positioned in the predetermined position relative to the tube body 1. In order to adjust the position of the suture needle guide 2 again, only pushing force for overcoming the elastic member needs to be exerted, and the positioning ball slides out of the concave part. Alternatively, in another method, the suture needle guide tube includes a positioning structure capable of locking and unlocking the tube body 1 and the suture needle guide 2. When the positioning structure is unlocked, the suture needle guide 2 is allowed to move axially, and when the suture needle guide moves axially to the predetermined position, the positioning structure is locked. In this case, a user handles the positioning structure to unlock the tube body 1 and the suture needle guide 2 from each other, so that the suture needle guide 2 can be moved axially. After the suture needle guide 2 is moved to the predetermined position, the user handles the positioning structure to lock the tube body 1 and the suture needle guide 2, so that the suture needle guide 2 can be held and positioned in the predetermined position.

Certainly, a plurality of types of positioning structures may be provided. For example, in a certain type of positioning structure, the positioning structure includes a positioning hole and a pin. For example, a plurality of positioning holes axially spaced apart from each other are formed on the tube body 1, and a pin hole is formed on the suture needle guide 2. After the suture needle guide 2 moves to the predetermined position, the pin passes through the pin hole, and is inserted into the positioning hole in the predetermined position, so that the suture needle guide 2 is held and positioned in the predetermined position. Alternatively, in another type of positioning structure, a positioning hole is formed on the suture needle guide 2, and a locking bolt may be thread-fitted in the positioning hole. In this manner, when the locking bolt is loosened, the suture needle guide 2 can move axially to the predetermined position. In the predetermined position, the locking bolt is tightened, so that an end portion of the locking bolt is pressed against an outer surface of the tube body 1, and the suture needle guide 2 is held and positioned in the predetermined position.

Additionally, in the suture needle guide tube 15, for the suture needle passing path on the tube body 1, a plurality of types thereof may be provided. For example, in a type, two protuberant plates circumferentially spaced apart from each other are formed on an outer surface on each of two sides opposite across the diameter of the tube body 1. Axially staggered holes are formed on the two protuberant plates on each side. An oblique distance between the holes of the two protuberant plates is the suture needle passing path. Alternatively, in another type, referring to FIG. 1 to FIG. 4, a plurality of pairs of suture needle passing ports are formed on a tube wall of the tube body 1, and each pair of suture needle passing ports includes one suture needle passing port 4 and the other suture needle passing port 4 that are axially spaced apart from each other and circumferentially staggered, so that an oblique distance between each pair of the two suture needle passing ports 4 forms the suture needle passing path. In this manner, the tube wall of the tube body 1 can be directly used to form the plurality of pairs of suture needle passing ports that are staggered, and the oblique distance between the two suture needle passing ports 4 of each pair of suture needle passing ports is the suture needle passing path. An oblique connecting line between the two suture needle passing ports 4 of each pair of suture needle passing ports may be substantially close to the central axis of the tube body 1, or located away from the central axis of the tube body 1, for example, located at an edge of the tube body 1.

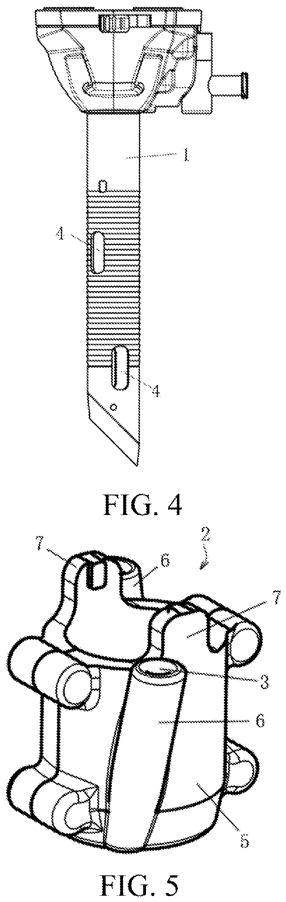

Additionally, in the suture needle guide tube 15, a plurality of types of suture needle guides 2 may be provided. For example, in a type of the suture needle guide 2, the suture needle guide 2 is a slide block, and the slide block is provided on the outer surface of the tube body 1, and is axially slidable. The suture needle guide channel 3 is formed on the slide block. Alternatively, in another type of the suture needle guide 2, referring to FIG. 5, the suture needle guide 2 includes a sleeve 5 and a plurality of oblique cylinders 6. The sleeve 5 is sleeved on the tube body 1. The plurality of oblique cylinders 6 are provided on an outer peripheral surface of the sleeve 5, and circumferentially spaced apart from each other. An internal channel of each oblique cylinder 6 is the suture needle guide channel 3. In this manner, referring to FIG. 1, the sleeve 5 only needs to be easily sleeved on the tube body 1. Additionally, annular mating and annular restriction between the sleeve 5 and the tube body 1 can also improve the stability of the sleeve 5, thereby further improving stability of a puncture performed by the suture needle.

Additionally, referring to FIG. 5, a protuberant piece 7 extends axially from an end surface of a sleeve wall at one end of the sleeve 5, and has a positioning hole, and a positioning column capable of being pressed against the tube body 1 is provided in the positioning hole. When the positioning column is pressed against the tube body 1, the sleeve 5 is held in the predetermined position, and when the positioning column disengages from the tube body 1, the sleeve 5 is allowed to move axially. The positioning column may be the locking bolt or pin described above.

A specific embodiment of the suture needle guide tube 15 is described below. The suture needle guide tube 15 includes a tube body 1 and a suture needle guide 2. A plurality of pairs of suture needle passing ports are formed on a tube wall of the tube body 1, and each pair of suture needle passing ports includes one suture needle passing port 4 and the other suture needle passing port 4 that are axially spaced apart from each other and circumferentially staggered. An oblique distance between each pair of the two suture needle passing ports 4 forms a suture needle passing path. The suture needle guide 2 includes a sleeve 5 and a plurality of oblique cylinders 6. The sleeve 5 is sleeved on the tube body 1. The plurality of oblique cylinders 6 are provided on an outer peripheral surface of the sleeve 5, and circumferentially spaced apart from each other. An internal channel of each oblique cylinder 6 is a suture needle guide channel 3. A protuberant piece 7 is formed on the sleeve 5, and is provided with a locking bolt. The sleeve 5 is capable of being adjusted to a predetermined position by means of axial movement along the tube body 1. After being tightened, the locking bolt is pressed against the tube body 1, so that the sleeve 5 is held in the predetermined position. In the predetermined position, each suture needle guide channel 3 communicates with the corresponding suture needle passing path in the predetermined position. In this manner, the suture needle can be inserted obliquely via the suture needle guide channel 3, then pass through the suture needle passing path, then be obliquely inserted into parietal tissue so as to enter an inner cavity.

In a second aspect, referencing to FIG. 1, FIG. 6, FIG. 10, and FIG. 13, a tissue suturing device provided in a specific embodiment of the present invention includes a sleeve tube main body 8, a handling member 9, and a suture arrangement member 11. The sleeve tube main body 8 is for being inserted into a wound to be sutured. The sleeve tube main body 8 includes a proximal handling end, a distal insertion end, and an axial channel between the proximal handling end and the distal insertion end. The sleeve tube main body 8 includes a plurality of suture needle paths located in different positions for guiding a suture needle to pass therethrough. The handling member 9 is provided at the proximal handling end, and includes a transmitting member 10 located in the axial channel. The suture arrangement member 11 is provided at the distal insertion end and for positioning a suture. The handling member 9 is capable of driving, by means of the transmitting member 10, the suture arrangement member 11 to switch between a folded position in which the distal insertion end and the suture arrangement member 11 are allowed to pass through the wound to be sutured of parietal tissue of an inner cavity and an unfolded position in which the suture is extended to and arranged on two sides of the wound to be sutured in the inner cavity. The suture needle path is for guiding the suture needle to pass through the parietal tissue to enter the inner cavity and for allowing the suture needle to exit with the suture.

In the tissue suturing device, the suture needle path on the sleeve tube main body 8 can effectively guide the suture needle to pass therethrough. In this manner, the suture needle can be prevented from shaking during a puncture, so that the suture needle can pass through the suture needle passing path stably, so as to pass through parietal tissue of an inner cavity and then enter the inner cavity. Additionally, the handling member 9 drives, by means of the transmitting member 10, the suture arrangement member 11 to change, in the inner cavity, from the folded position to the unfolded position of spanning the wound to be sutured, so that the suture is accurately extended to and arranged on two sides of the wound to be sutured in the inner cavity. After the suture needle having entered the inner cavity holds the suture, effectively guided by the suture needle path on the sleeve tube main body 8, the suture needle with the suture can stably pass through the parietal tissue and then exit. Therefore, the tissue suturing device not only can guide a suture needle to puncture accurately in a predetermined direction to prevent a deviated puncture caused by shaking of the suture needle, but also can accurately arrange a suture in a desired position, so that the suture can be led out after the suture needle punctures once, thereby significantly reducing injury to tissue of a wound to be sutured of a patient.

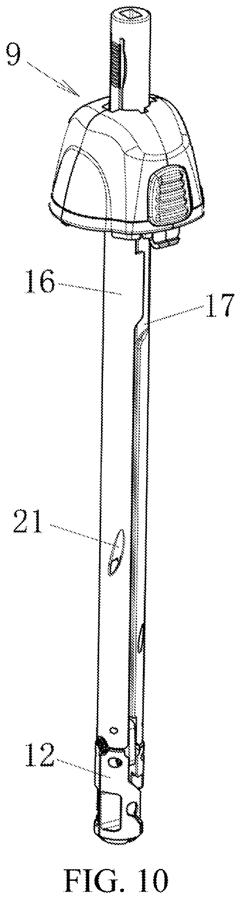

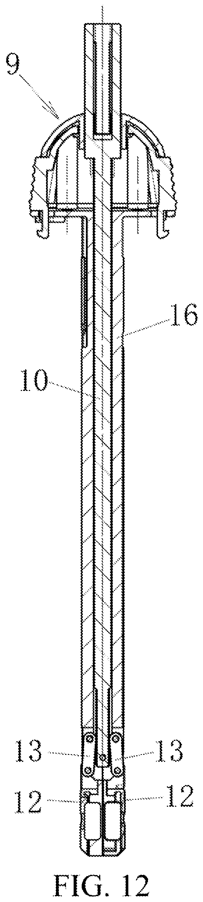

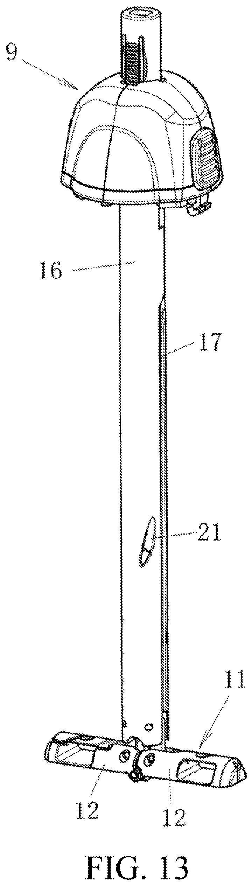

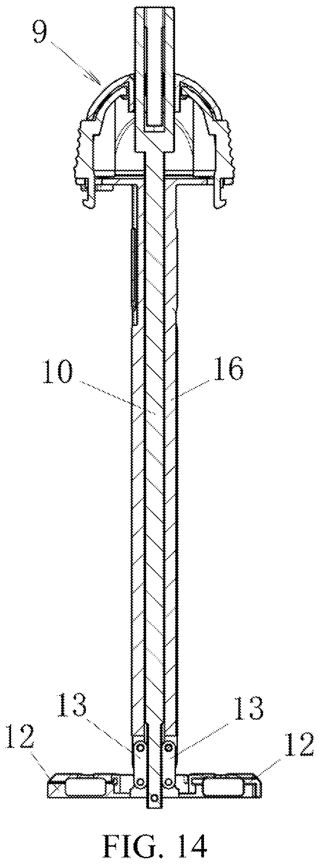

In this tissue suturing device, a plurality of types of suture arrangement members 11 may be provided. For example, in a type of the suture arrangement member 11, the suture arrangement member 11 may include a main body and two opposite movable telescopic suture arrangement rods. When the two telescopic suture arrangement rods move to a retracted position, the distal insertion end and the suture arrangement member 11 can enter and exit the wound to be sutured. In the inner cavity, after moving and extending, the two telescopic suture arrangement rods can span two sides of the wound to be sutured, so as to cause the suture to be accurately extended to and arranged on the two sides of the wound to be sutured. Alternatively, in another type of the suture arrangement member 11, referring to FIG. 10, FIG. 13, and FIG. 14, the suture arrangement member 11 includes two suture arrangement wings 12. Respective ends of the two suture arrangement wings 12 are hinge-connected to the transmitting member 10. A linkage 13 is hinge-connected between each suture arrangement wing and the distal insertion end. The transmitting member 10 is capable of driving the two suture arrangement wings 12 to fold and unfold when driven by the handling member 9 to move telescopically axially. In this manner, since the two suture arrangement wings 12 can rotate, an occupied space is reduced, and injury to the wound to be sutured caused by entering or exiting the wound to be sutured can be reduced as much as possible. In FIG. 12, when the handling member 9 drives the transmitting member 10 to move downwards, the two suture arrangement wings 12 can be driven by the two linkages 13 to rotate, so as to change from the folded position shown in FIG. 10 to the unfolded position shown in FIG. 13, so that the two suture arrangement wings 12 span the two sides of the wound to be sutured, so as to cause the suture to be accurately extended to and arranged on the two sides of the wound to be sutured. In this manner, after the suture needle respectively punctures into the inner cavity obliquely from the two sides, the suture 22 can be respectively pulled to the outside from the two suture arrangement wings 12, so as to suture the wound to be sutured. Additionally, the transmitting member 10 may be one single rod or other activation force transmitting members connected to each other.

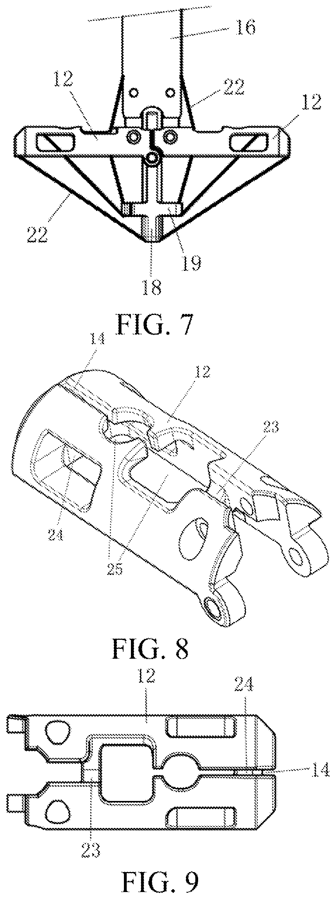

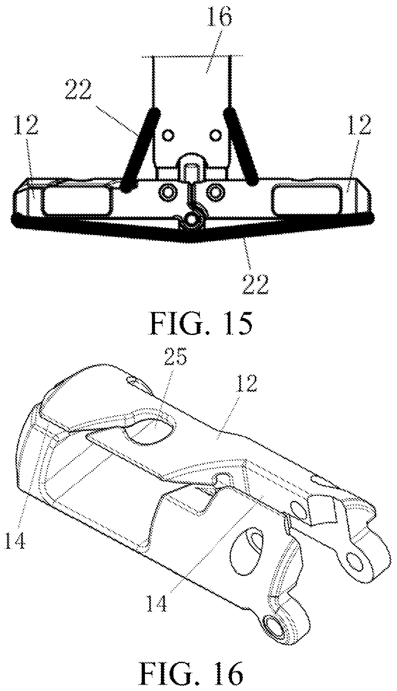

Additionally, in order for the suture needle to hook or hold the suture easily, with reference to a type of suture arrangement wing 12 shown in FIG. 8, and another type of suture arrangement wing 12 shown in FIG. 16, the suture arrangement wing 12 is provided with a suture needle suture-holding port 25. For example, the suture arrangement wing 12 shown in FIG. 16 has one suture needle suture-holding port 25. The suture arrangement wing 12 shown in FIG. 12 has two suture needle suture-holding ports 25 corresponding to suture needles inserted in different inclination angles. In this manner, after the suture needle is obliquely inserted into the suture needle suture-holding port 25, the suture can be easily hooked or held.

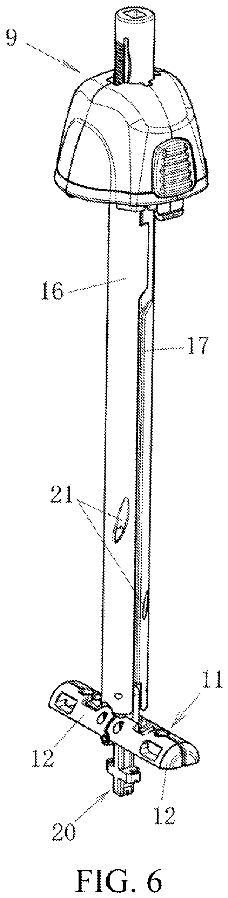

Additionally, in the tissue suturing device, with reference to FIG. 6 and FIG. 7, the suture arrangement member 11 includes a cross 20 having a vertical rod 18 and a transverse rod 19. One end of the vertical rod 18 is connected to the transmitting member 10, and the other end of the vertical rod 18 is for allowing the suture to bypass the same. Two ends of the transverse rod 19 are respectively for allowing the suture to pass therethrough. In this manner, winding of the suture 22 by means of the cross 20 and the two suture arrangement wings 12 enables the suture needle to easily hold the suture 22 so as to pull the suture 22 to the outside to suture the wound to be sutured. For example, referring to FIG. 7, FIG. 8, and FIG. 9, after moving downwards from the left side, the suture is then wound on a suture support column 23 of the suture arrangement wing 12 on the left side, is then wound downwards in a left hole of the transverse rod 19, then extends upwards to be wound on a suture bypassing column 24 of the suture arrangement wing 12 on the left side, then extends downwards obliquely to be wound in a receiving recess on a lower end of the vertical rod 18, is then subjected, on the right side, to winding of the left side type, and then extends upwards.

Additionally, in order for the two suture arrangement wings 12 to change from the folded position to the unfolded position more easily to improve easiness of handling, an elastic member is provided in a position where the respective ends of the two suture arrangement wings 12 and the transmitting member 10 are hinge-connected to each other. The elastic member may be an elastic sleeve or a torsional spring, and is capable of accumulating force when the two suture arrangement wings are in the folded position, so as to apply biasing force to the two suture arrangement wings 12 to cause the same to rotate towards the unfolded position. In this manner, when applying force, the elastic member drives the two suture arrangement wings 12 to extend rapidly.

Additionally, the suture 22 may be affixed to the suture arrangement member 11. Alternatively, in order to further improve reliability of pre-positioning of the suture 22, referring to FIG. 8 and FIG. 16, a suture accommodating gap 14 for pre-positioning the suture and allowing the suture to be hooked out by the suture needle is formed on the suture arrangement member 11. In this manner, the suture 22 can be pre-positioned in the suture accommodating gap 14, so that when the suture arrangement member 11 carrying the suture passes through the wound to be sutured, the suture does not separate accidentally from the suture arrangement member 11.

Additionally, the tissue suturing device includes a suture needle (not shown) capable of holding the suture. The suture needle is capable of passing through the suture needle path to pass through the parietal tissue to enter the inner cavity and exiting with the suture. That is, after a front end of the suture needle is obliquely transmitted into the inner cavity, the front end of the suture needle hooks or holds, on the suture arrangement member 11 in the unfolded position, the suture, so that when exiting along the entry path, the suture needle can pull the suture to the outside.

Additionally, it should be noted that in the tissue suturing device, a plurality of types of sleeve tube main bodies 8 may be provided. For example, the sleeve tube main body 8 may be a separate tube, and the tube may include a plurality of suture needle paths located in different positions for guiding the suture needle to pass therethrough. Alternatively, with reference to FIG. 1, FIG. 3, FIG. 6, and FIG. 13, the sleeve tube main body 8 includes a suture needle guide tube 15 and a handling tube 16. The suture needle guide tube 15 includes a suture needle path. The handling tube 16 is provided in an internal axial channel of the suture needle guide tube 15. The handling member 9 is provided at a proximal handling end of the handling tube 16. The transmitting member 10 is located in an axial channel of the handling tube 16. The suture arrangement member 11 is provided at a distal insertion end of the handling tube 16. In this manner, when used, the handling tube 16 can be inserted into the suture needle guide tube 15 so as to form an annular gap therebetween, and the suture needle can enter or exit by means of the suture needle path on the suture needle guide tube 15. For example, the suture needle can pass through the annular gap. Alternatively, if the handling tube 16 is likely to interfere with the suture needle, an oblique suture passing hole 21 can be formed on the handling tube 16 as shown in FIG. 6, so that the suture needle can pass through the suture passing hole 21, thereby preventing the handling tube 16 from interfering with the suture needle.

Additionally, with reference to FIG. 6, FIG. 10, and FIG. 13, a plurality of suture receiving recesses 17 circumferentially spaced apart from each other and extending axially are formed on an outer side surface of the handling tube 16. In this manner, the suture can be pre-positioned in the suture receiving recess 17. By means of restriction performed by the suture receiving recess 17, the suture can be prevented from being dislocated due to looseness, so that the suture needle can more easily pull the suture to the outside.

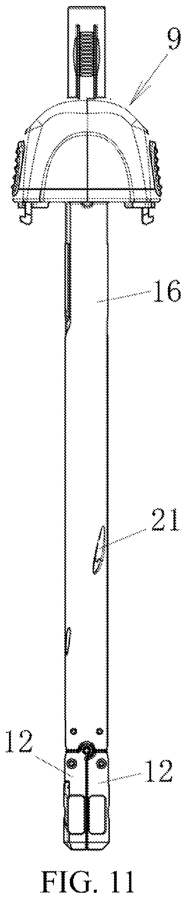

Additionally, in order to facilitate arrangement and pulling of the suture, the handling member 9 includes suture leading holes spaced apart from each other, so as to allow the suture to pass through the suture leading hole on one side, then extend axially along the handling tube 16, for example, along the suture receiving recess 17, be wound around the suture arrangement member 11, then return along the handling tube 16, and extend out from the suture leading-out hole on the other side. In this manner, for example in FIG. 11, the suture can be perpendicularly pulled between the handling member 9 and the handling tube 16, thereby preventing the suture from sticking.

Additionally, it should be further noted herein that the suture needle guide tube 15 described in the second aspect of the present invention may be a separate tube, and no other suture needle guide component is provided on the outer surface of the tube. Alternatively, with reference to FIG. 1, the suture needle guide tube 15 in the second aspect of the present invention is the suture needle guide tube 15 according to the first aspect, and the suture needle passing path and the suture needle guide channel 3 form the suture needle path. In this manner, as described above, effective guiding performed by the suture needle guide channel 3 prevents a suture needle from shaking during a puncture, so that the suture needle can pass through the suture needle passing path stably obliquely, so as to pass through parietal tissue of an inner cavity and then enter the inner cavity. Additionally, after the suture needle holds the suture, effectively guided by the suture needle guide channel 3, the suture needle with the suture can stably pass through the parietal tissue and then exit, so that the suture is guided to pass through the wound to be sutured so as to suture the same. Therefore, the suture needle guide tube 15 in the first aspect can guide a suture needle to puncture accurately in a predetermined direction, and a deviated puncture caused by shaking of the suture needle is avoided, thereby reducing injury to tissue of a wound to be sutured of a patient.

Those skilled in the art should understand that the above embodiments are all exemplary instead of limiting. Different technical features appearing in different embodiments may be combined to achieve benefits. After studying the accompanying drawings, description, and claims, those skilled in the art should be able to understand and implement the disclosed embodiments and other variations of the embodiments. Any reference numerals in the claims should not be construed as limiting the scope of protection. Certain technical features appearing in different dependent claims do not mean that these technical features could not be combined to achieve benefits.

## Claims

1. A suture needle guide tube (15), comprising:
a tube body (1), for being inserted into a wound to be sutured, the tube body (1) comprising an internal axial channel for arranging a handling tube (16) provided with a suture arrangement member (11) and a handling member (9) therein, and the tube body (1) being provided with a plurality of suture needle passing paths located in different positions for guiding a suture needle to pass therethrough; and
a suture needle guide (2), provided with a plurality of suture needle guide channels (3) located in different positions;
wherein the suture needle guide (2) is provided on the tube body (1), and each suture needle guide channel (3) respectively communicates with a corresponding one of the plurality of suture needle passing paths so as to guide the suture needle to move out of the suture needle guide channel (3) and then pass through the suture needle passing path to enter an inner cavity and to allow the suture needle to exit with a suture, and **characterized in that**:
the suture needle guide (2) is capable of being adjusted to a plurality of predetermined positions by means of axial movement along the tube body (1) so that each of the suture needle guide channels (3) communicates with a corresponding one of the plurality of suture needle passing paths in a corresponding one of the plurality of predetermined positions.

2. The suture needle guide tube according to claim 1, comprising a positioning structure capable of locking and unlocking the tube body (1) and the suture needle guide (2), wherein when the positioning structure is unlocked, the suture needle guide (2) is allowed to move axially, and when the suture needle guide (2) moves axially to the predetermined position, the positioning structure is locked.

3. The suture needle guide tube according to claim 1 or 2, wherein a plurality of pairs of suture needle passing ports are formed on a tube wall of the tube body (1), and each pair of suture needle passing ports comprises one suture needle passing port (4) and the other suture needle passing port (4) that are axially spaced apart from each other and circumferentially staggered, an oblique distance between each pair of the two suture needle passing ports (4) forming the suture needle passing path.

4. The suture needle guide tube according to claim 3, wherein the suture needle guide (2) comprises a sleeve (5) and a plurality of oblique cylinders (6), the sleeve (5) being sleeved on the tube body (1), and the plurality of oblique cylinders (6) being provided on an outer peripheral surface of the sleeve (5) and circumferentially spaced apart from each other, wherein an internal channel of each oblique cylinder (6) is the suture needle guide channel (3).

5. The suture needle guide tube according to claim 4, wherein a protuberant piece (7) extends axially from an end surface of a sleeve wall at one end of the sleeve (5), and has a positioning hole, and a positioning column capable of being pressed against the tube body (1) is provided in the positioning hole, wherein when the positioning column is pressed against the tube body (1), the sleeve (5) is held in a predetermined position, and when the positioning column disengages from the tube body (1), the sleeve (5) is allowed to move axially.

6. A tissue suturing device, comprising:
a sleeve tube main body (8), for being inserted into a wound to be sutured, the sleeve tube main body (8) including the suture needle guide tube (15) according to claim 1, the sleeve tube main body (8) comprising a proximal handling end, and a distal insertion end, wherein the internal axial channel extends between the proximal handling end and the distal insertion end, and the sleeve tube main body (8) comprising a plurality of suture needle paths located in different positions for guiding a suture needle to pass therethrough;
the handling member (9), provided at the proximal handling end and comprising a transmitting member (10) located in the axial channel; and
the suture arrangement member (11), provided at the distal insertion end and for positioning a suture;
wherein the handling member (9) is capable of driving, by means of the transmitting member (10), the suture arrangement member (11) to switch between a folded position in which the distal insertion end and the suture arrangement member (11) are allowed to pass through the wound to be sutured of parietal tissue of an inner cavity and an unfolded position in which the suture is extended to and arranged on two sides of the wound to be sutured in the inner cavity;
wherein the suture needle path is for guiding the suture needle to pass through the parietal tissue to enter the inner cavity and for allowing the suture needle to exit with the suture.

7. The tissue suturing device according to claim 6, wherein the suture arrangement member (11) comprises two suture arrangement wings (12), wherein respective ends of the two suture arrangement wings (12) are hinge-connected to the transmitting member (10), and a linkage (13) is hinge-connected between each of the suture arrangement wings and the distal insertion end, the transmitting member (10) being capable of driving the two suture arrangement wings (12) to fold and unfold when driven by the handling member (9) to move telescopically axially.

8. The tissue suturing device according to claim 7, wherein the suture arrangement member (11) comprises a cross (20) having a vertical rod (18) and a transverse rod (19), wherein one end of the vertical rod (18) is connected to the transmitting member (10), and the other end of the vertical rod (18) is for allowing the suture to bypass the same, two ends of the transverse rod (19) being respectively for allowing the suture to pass therethrough.

9. The tissue suturing device according to claim 7 or claim 8, wherein an elastic member is provided in a position where the respective ends of the two suture arrangement wings (12) and the transmitting member (10) are hinge-connected to each other, and the elastic member is capable of accumulating force when the two suture arrangement wings (12) are in the folded position, so as to apply biasing force to the two suture arrangement wings (12) to cause the same to rotate towards the unfolded position.

10. The tissue suturing device according to any one of claims 6 - 9, wherein a suture accommodating gap (14) for pre-positioning the suture and allowing the suture to be hooked out by the suture needle is formed on the suture arrangement member (11).

11. The tissue suturing device according to any one of claims 6 - 10, comprising the suture needle capable of holding the suture, wherein the suture needle is capable of passing through the suture needle path to pass through the parietal tissue to enter the inner cavity and exiting with the suture.

12. The tissue suturing device according to any one of claims 6 - 11, wherein the sleeve tube main body (8) further comprises a handling tube (16), wherein the suture needle guide tube (15) comprises the suture needle path, the suture needle path being formed by the suture needle passing path and the suture needle guide channel (3), wherein the handling tube (16) is provided in an internal axial channel of the suture needle guide (15), the handling member (9) is provided at a proximal handling end of the handling tube (16), the transmitting member (10) is located in an axial channel of the handling tube (16), and the suture arrangement member (11) is provided at a distal insertion end of the handling tube (16).

13. The tissue suturing device according to claim 12, wherein a plurality of suture receiving recesses (17) circumferentially spaced apart from each other and extending axially are formed on an outer side surface of the handling tube (16).

14. The tissue suturing device according to claim 12 or claim 13, wherein the handling member (9) comprises suture leading holes spaced apart from each other, so as to allow the suture to pass through the suture leading hole on one side, then extend axially along the handling tube (16), be wound around the suture arrangement member (11), then return along the handling tube (16), and extend out from the suture leading-out hole on the other side.

## Patentansprüche

1. Führungsrohr (15) für eine Nahtnadel, umfassend:
einen Rohrkörper (1) zum Einführen in eine zu nähende Wunde, wobei der Rohrkörper (1) einen inneren axialen Kanal zum Anordnen eines Handhabungsrohrs (16) umfasst, das mit einem Nahtmaterialanordnungselement (11) und einem Handhabungselement (9) darin versehen ist, und wobei der Rohrkörper (1) mit einer Vielzahl von an unterschiedlichen Positionen angeordneten Nahtnadeldurchgangspfaden zum Durchführen einer Nahtnadel versehen ist; und
eine Nahtnadelführung (2), die mit einer Vielzahl von Nahtnadelführungskanälen (3) ausgestattet ist, die sich an unterschiedlichen Positionen befinden;
wobei die Nahtnadelführung (2) am Rohrkörper (1) vorgesehen ist, und jeder Nahtnadelführungskanal (3) jeweils mit einem entsprechenden einen der Vielzahl von Nahtnadeldurchgangspfaden in Verbindung steht, um die Nahtnadel aus dem Nahtnadelführungskanal (3) herauszuführen und dann durch den Nahtnadeldurchgangspfad in einen Innenhohlraum einzutreten und es der Nahtnadel zu ermöglichen, mit einem Nahtmaterial herauszukommen, und
**dadurch gekennzeichnet, dass:**
die Nahtnadelführung (2) in der Lage ist, durch axiale Bewegung entlang des Rohrkörpers (1) auf eine Vielzahl von vorbestimmten Positionen eingestellt zu werden, so dass jeder der Nahtnadelführungskanäle (3) mit einem entsprechenden einen der Vielzahl von Nahtnadeldurchgangspfaden in einer entsprechenden der Vielzahl von vorbestimmten Positionen kommuniziert.

2. Nahtnadelführungsrohr nach Anspruch 1, umfassend eine Positionierungsstruktur, die in der Lage ist, den Rohrkörper (1) und die Nahtnadelführung (2) zu verriegeln und zu entriegeln, wobei, wenn die Positionierungsstruktur entriegelt ist, die Nahtnadelführung (2) sich axial bewegen kann, und wenn sich die Nahtnadelführung (2) axial in die vorbestimmte Position bewegt, die Positionierungsstruktur verriegelt ist.

3. Nahtnadelführungsrohr nach Anspruch 1 oder 2, wobei eine Vielzahl von Paaren von Nahtnadeldurchgangsöffnungen an einer Rohrwand des Rohrkörpers (1) ausgebildet sind, und jedes Paar von Nahtnadeldurchgangsöffnungen eine Nahtnadeldurchgangsöffnung (4) und die andere Nahtnadeldurchgangsöffnung (4) umfasst, die axial voneinander beabstandet und in Umfangsrichtung versetzt sind, wobei ein schräger Abstand zwischen jedem Paar der zwei Nahtnadeldurchgangsöffnungen (4) den Nahtnadeldurchgangspfad bildet.

4. Nahtnadelführungsrohr nach Anspruch 3, wobei die Nahtnadelführung (2) eine Hülse (5) und eine Vielzahl von schrägen Zylindern (6) umfasst, wobei die Hülse (5) auf den Rohrkörper (1) aufgesteckt ist, und die Vielzahl von schrägen Zylindern (6) an einer Außenumfangsfläche der Hülse (5) vorgesehen und in Umfangsrichtung voneinander beabstandet sind, wobei ein Innenkanal jedes schrägen Zylinders (6) der Nahtnadelführungskanal (3) ist.

5. Nahtnadelführungsrohr nach Anspruch 4, wobei sich ein hervorstehendes Stück (7) axial von einer Endoberfläche einer Hülsenwand an einem Ende der Hülse (5) erstreckt, und ein Positionierungsloch aufweist, und in dem Positionierungsloch eine Positionierungssäule vorgesehen ist, die gegen den Rohrkörper (1) gedrückt werden kann, wobei die Hülse (5) in einer vorbestimmten Position gehalten wird, wenn die Positionierungssäule gegen den Hülsenkörper (1) gedrückt wird, und sich die Hülse (5) axial bewegen kann, wenn sich die Positionierungssäule vom Rohrkörper (1) löst.

6. Gewebenähvorrichtung, umfassend:
einen Hülsenrohrhauptkörper (8) zum Einführen in eine zu nähende Wunde, wobei der Hülsenrohrhauptkörper (8) das Nahtnadelführungsrohr (15) nach Anspruch 1 einschließt, wobei der Hülsenrohrhauptkörper (8) ein proximales Handhabungsende und ein distales Einführende umfasst, wobei sich der innere axiale Kanal zwischen dem proximalen Handhabungsende und dem distalen Einführende erstreckt, und wobei der Hülsenrohrhauptkörper (8) eine Vielzahl von Nahtnadelpfaden umfasst, die sich an unterschiedlichen Positionen befinden, um eine Nahtnadel hindurchzuführen;
das Handhabungselement (9), das am proximalen Handhabungsende vorgesehen ist und ein Übertragungselement (10) umfasst, das sich im axialen Kanal befindet; und
das Nahtmaterialanordnungselement (11), das am distalen Einführende vorgesehen ist und zum Positionieren eines Nahtmaterials dient;
wobei das Handhabungselement (9) mittels des Übertragungselements (10) in der Lage ist, das Nahtmaterialanordnungselement (11) anzutreiben, um zwischen einer gefalteten Position, in der das distale Einführende und das Nahtmaterialanordnungselement (11) durch die zu nähende Wunde des parietalen Gewebes eines Innenhohlraums hindurchgeführt werden können, und einer entfalteten Position, in der das Nahtmaterial zu bis zu zwei Seiten der zu nähenden Wunde in dem Innenhohlraum reicht und auf diesen angeordnet ist, umzuschalten;
wobei der Nahtnadelpfad dazu dient, die Nahtnadel durch das parietale Gewebe zu führen, um in den Innenhohlraum einzudringen, und um der Nahtnadel zu ermöglichen, mit dem Nahtmaterial auszutreten.

7. Gewebenähvorrichtung nach Anspruch 6, wobei das Nahtmaterialanordnungselement (11) zwei Nahtmaterialanordnungsflügel (12) umfasst, wobei die jeweiligen Enden der beiden Nahtmaterialanordnungsflügel (12) scharnierartig mit dem Übertragungselement (10) verbunden sind und eine Verbindung (13) scharnierartig zwischen jedem der Nahtmaterialanordnungsflügel und dem distalen Einführende verbunden ist, wobei das Übertragungselement (10) in der Lage ist, die beiden Nahtmaterialanordnungsflügel (12) anzutreiben, um sie zu falten und zu entfalten, wenn es durch das Handhabungselement (9) angetrieben wird, um sich teleskopartig axial zu bewegen.

8. Gewebenähvorrichtung nach Anspruch 7, wobei das Nahtmaterialanordnungselement (11) ein Kreuz (20) mit einer vertikalen Stange (18) und einer Querstange (19) umfasst, wobei ein Ende der vertikalen Stange (18) mit dem Übertragungselement (10) verbunden ist und das andere Ende der vertikalen Stange (18) dazu dient, dem Nahtmaterial die Umgehung desselben zu ermöglichen, während zwei Enden der Querstange (19) jeweils dazu dienen, dem Nahtmaterial den Durchgang dort hindurch zu ermöglichen.

9. Gewebenähvorrichtung nach Anspruch 7 oder Anspruch 8, wobei ein elastisches Element an einer Stelle vorgesehen ist, an der die jeweiligen Enden der beiden Nahtmaterialanordnungsflügel (12) und des Übertragungselements (10) scharnierartig miteinander verbunden sind, und das elastische Element in der Lage ist, Kraft zu akkumulieren, wenn sich die beiden Nahtmaterialanordnungsflügel (12) in der gefalteten Position befinden, um so eine Vorspannkraft auf die beiden Nahtmaterialanordnungsflügel (12) auszuüben, um diese zu veranlassen, sich in die entfaltete Position zu drehen.

10. Gewebenähvorrichtung nach einem der Ansprüche 6 bis 9, wobei an dem Nahtmaterialanordnungselement (11) ein Nahtmaterialaufnahmespalt (14) zum Vorpositionieren des Nahtmaterials und zum Ermöglichen des Aushakens des Nahtmaterials durch die Nahtnadel ausgebildet ist.

11. Gewebenähvorrichtung nach einem der Ansprüche 6 bis 10, die die Nahtnadel umfasst, die in der Lage ist, das Nahtmaterial zu halten, wobei die Nahtnadel durch den Nahtnadelpfad geführt werden kann, um durch das parietale Gewebe zu gelangen, in den Innenhohlraum einzutreten und mit dem Nahtmaterial wieder auszutreten.

12. Gewebenähvorrichtung nach einem der Ansprüche 6 bis 11, wobei der Hülsenrohrhauptkörper (8) ferner ein Handhabungsrohr (16) umfasst, wobei das Nahtnadelführungsrohr (15) den Nahtnadelpfad umfasst, der durch den Nahtnadeldurchgangspfad und den Nahtnadelführungskanal (3) gebildet wird, wobei das Handhabungsrohr (16) in einem inneren axialen Kanal der Nahtnadelführung (15) vorgesehen ist, das Handhabungselement (9) an einem proximalen Handhabungsende des Handhabungsrohrs (16) vorgesehen ist, das Übertragungselement (10) in einem axialen Kanal des Handhabungsrohrs (16) angeordnet ist, und das Nahtmaterialanordnungselement (11) an einem distalen Einführende des Handhabungsrohrs (16) vorgesehen ist.

13. Gewebenähvorrichtung nach Anspruch 12, wobei auf einer Außenseite des Handhabungsrohrs (16) eine Vielzahl von Nahtmaterialaufnahmeaussparungen (17) ausgebildet sind, die in Umfangsrichtung voneinander beabstandet sind und sich axial erstrecken.

14. Gewebenähvorrichtung nach Anspruch 12 oder 13, wobei das Handhabungselement (9) Nahtmaterialführungslöcher aufweist, die im Abstand voneinander angeordnet sind, so dass das Nahtmaterial auf der einen Seite durch das Nahtmaterialführungsloch hindurchtreten, sich dann axial entlang des Handhabungsrohrs (16) erstrecken, um das Nahtmaterialanordnungselement (11) gewickelt werden, dann entlang des Handhabungsrohrs (16) zurückkehren und aus dem Nahtmaterialführungsloch auf der anderen Seite heraustreten kann.

## Revendications

1. Tube de guidage d'aiguille de suture (15), comprenant :
un corps de tube (1), destiné à être inséré dans une plaie à suturer, le corps de tube (1) comprenant un canal axial interne destiné à agencer un tube de manipulation (16) pourvu d'un élément d'agencement de suture (11) et d'un élément de manipulation (9) à l'intérieur de celui-ci, et le corps de tube (1) étant pourvu d'une pluralité de voies de passage d'aiguille de suture situées dans des positions différentes destinées à guider une aiguille de suture à passer à travers de celle-ci ; et
un guide d'aiguille de suture (2), pourvu d'une pluralité de canaux de guidage d'aiguille de suture (3) situés dans des positions différentes ;
dans lequel le guide d'aiguille de suture (2) est fourni sur le corps de tube (1), et chaque canal de guidage d'aiguille de suture (3) communique respectivement avec une voie correspondante parmi la pluralité de voies de passage d'aiguille de suture de façon à guider l'aiguille de suture à sortir du canal de guidage d'aiguille de suture (3) puis à passer à travers la voie de passage d'aiguille de suture pour entrer dans une cavité interne et à permettre à l'aiguille de suture de sortir avec une suture, et **caractérisé en ce que :**
le guide d'aiguille de suture (2) est susceptible de s'ajuster à une pluralité de positions prédéterminées au moyen d'un mouvement axial le long du corps de tube (1) de sorte que chacun des canaux de guidage d'aiguille de suture (3) communique avec une voie correspondante parmi la pluralité de voies de passage d'aiguille de suture dans une voie correspondante parmi la pluralité de positions prédéterminées.

2. Tube de guidage d'aiguille de suture selon la revendication 1, comprenant une structure de positionnement susceptible de verrouiller et de déverrouiller le corps de tube (1) et le guide d'aiguille de suture (2), dans lequel lorsque la structure de positionnement est déverrouillée, le guide d'aiguille de suture (2) est autorisé à se déplacer axialement, et lorsque le guide d'aiguille de suture (2) se déplace axialement jusqu'à la position prédéterminée, la structure de positionnement est verrouillée.

3. Tube de guidage d'aiguille de suture selon la revendication 1 ou 2, dans lequel une pluralité de paires d'orifices de passage d'aiguille de suture sont formées sur une paroi de tube du corps de tube (1), et chaque paire d'orifices de passage d'aiguille de suture comprend un port de passage d'aiguille de suture (4) et l'autre orifice de passage d'aiguille de suture (4) qui sont espacés axialement l'un de l'autre et disposé en quinconce circonférentiellement, une distance oblique entre chaque paire des deux orifices de passage d'aiguille de suture (4) formant la voie de passage de l'aiguille de suture.

4. Tube de guidage d'aiguille de suture selon la revendication 3, dans lequel le guide d'aiguille de suture (2) comprend un manchon (5) et une pluralité de cylindres obliques (6), le manchon (5) étant manchonné sur le corps de tube (1), et la pluralité de cylindres obliques (6) étant fourni sur une surface périphérique externe du manchon (5) et espacés circonférentiellement les uns des autres, dans lequel un canal interne de chaque cylindre oblique (6) est le canal de guidage d'aiguille de suture (3).

5. Tube de guidage d'aiguille de suture selon la revendication 4, dans lequel une pièce protubérante (7) s'étend axialement à partir d'une surface d'extrémité d'une paroi de manchon au niveau d'une extrémité du manchon (5), et a un trou de positionnement, et une colonne de positionnement susceptible d'être pressée contre le corps de tube (1) est fournie dans le trou de positionnement, dans lequel lorsque la colonne de positionnement est pressée contre le corps de tube (1), le manchon (5) est maintenu dans une position prédéterminée, et lorsque la colonne de positionnement se désengage du corps de tube (1), le manchon (5) est autorisé à se déplacer axialement.

6. Dispositif de suture de tissu comprenant :
un corps principal de tube de manchon (8), destiné à être inséré dans une plaie à suturer, le corps principal de tube de manchon (8) comportant le tube de guidage d'aiguille de suture (15) selon la revendication 1, le corps principal de tube de manchon (8) comprenant une extrémité de manipulation proximale, et une extrémité d'insertion distale, dans lequel le canal axial interne s'étend entre l'extrémité de manipulation proximale et l'extrémité d'insertion distale, et le corps principal de tube de manchon (8) comprenant une pluralité de voies d'aiguille de suture situées dans différentes positions destinées à guider une aiguille de suture à passer à travers celle-ci ;
l'élément de manipulation (9), fourni au niveau de l'extrémité de manipulation proximale et comprenant un élément de transmission (10) situé dans le canal axial ; et
l'élément d'agencement de suture (11), fourni au niveau de l'extrémité d'insertion distale et destiné à positionner une suture ;
dans lequel l'élément de manipulation (9) est susceptible d'entraîner, au moyen de l'élément de transmission (10), l'élément d'agencement de suture (11) pour passer d'une position repliée dans laquelle l'extrémité d'insertion distale et l'élément d'agencement de suture (11) peuvent passer à travers la plaie à suturer du tissu pariétal d'une cavité interne à une position dépliée dans laquelle la suture est étendue et agencée sur deux côtés de la plaie à suturer dans la cavité interne ;
dans lequel la voie d'aiguille de suture est destinée à guider l'aiguille de suture à travers le tissu pariétal pour pénétrer dans la cavité interne et destinée à permettre à l'aiguille de suture de sortir avec le fil de suture.

7. Dispositif de suture de tissu selon la revendication 6, dans lequel l'élément d'agencement de suture (11) comprend deux ailes d'agencement de suture (12), les extrémités respectives des deux ailes d'agencement de suture (12) étant reliées par une charnière à l'élément de transmission (10), et une liaison (13) est reliée par une charnière entre chacune des ailes d'agencement de suture et l'extrémité d'insertion distale, l'élément de transmission (10) étant susceptible d'entraîner les deux ailes d'agencement de suture (12) à se plier et à se déplier lorsqu'elles sont entraînées par l'élément de manipulation (9) à se déplacer axialement de manière télescopique.

8. Dispositif de suture de tissu selon la revendication 7, dans lequel l'élément d'agencement de suture (11) comprend une croix (20) ayant une tige verticale (18) et une tige transversale (19), dans lequel une extrémité de la tige verticale (18) est reliée à l'élément de transmission (10), et l'autre extrémité de la tige verticale (18) est destinée à permettre à la suture de la contourner celui-ci, deux extrémités de la tige transversale (19) étant respectivement destinées à permettre à la suture de passer à travers celui-ci.

9. Dispositif de suture de tissu selon la revendication 7 ou la revendication 8, dans lequel un élément élastique est fourni dans une position où les extrémités respectives des deux ailes d'agencement de suture (12) et de l'élément de transmission (10) sont reliées par une charnière l'une à l'autre, et l'élément élastique est susceptible d'accumuler une force lorsque les deux ailes d'agencement de suture (12) sont en position pliée, de façon à appliquer une force de sollicitation aux deux ailes d'agencement de suture (12) pour les faire tourner vers la position dépliée.

10. Dispositif de suture de tissu selon l'une quelconque des revendications 6 à 9, dans lequel un trou de logement de suture (14) destiné à prépositionner la suture et à permettre à la suture d'être accrochée par l'aiguille de suture est formé sur l'élément d'agencement de suture (11).

11. Dispositif de suture de tissu selon l'une quelconque des revendications 6 à 10, comprenant l'aiguille de suture susceptible de tenir la suture, dans lequel l'aiguille de suture est capable de passer à travers la voie d'aiguille de suture afin de passer à travers le tissu pariétal afin de pénétrer dans la cavité interne et de sortir avec la suture.

12. Dispositif de suture de tissu selon l'une quelconque des revendications 6 à 11, dans lequel le corps principal de tube de manchon (8) comprend en outre un tube de manipulation (16), dans lequel le tube de guidage d'aiguille de suture (15) comprend la voie d'aiguille de suture, la voie d'aiguille de suture étant formé par la voie de passage d'aiguille de suture et le canal de guidage d'aiguille de suture (3), dans lequel le tube de manipulation (16) est fourni dans un canal axial interne du guide d'aiguille de suture (15), l'élément de manipulation (9) est fourni au niveau d'une extrémité de manipulation proximale du tube de manipulation (16), l'élément de transmission (10) est situé dans un canal axial du tube de manipulation (16), et l'élément d'agencement de suture (11) est fourni au niveau d'une extrémité d'insertion distale du tube de manipulation (16).

13. Dispositif de suture de tissu selon la revendication 12, dans lequel une pluralité de cavités de réception de suture (17) espacés circonférentiellement les uns des autres et s'étendant axialement sont formées sur une surface latérale externe du tube de manipulation (16).

14. Dispositif de suture de tissu selon la revendication 12 ou la revendication 13, dans lequel l'élément de manipulation (9) comprend des trous d'orientation de suture espacés les uns des autres, de façon à permettre à la suture de passer à travers le trou d'orientation de suture d'un côté, puis de s'étendre axialement le long du tube de manipulation (16), d'être enroulée autour de l'élément d'agencement de suture (11), puis de revenir le long du tube de manipulation (16), et de s'étendre hors du trou d'orientation de sortie de suture de l'autre côté.
